# EUROPEAN PATENT SPECIFICATION

(11) **EP 0 415 978 B1**
(45) Date of publication and mention of the grant of the patent: **10.04.1996**
(21) Application number: 89905797.0
(22) Date of filing: 24.05.1989
(51) Int. Cl.: C12Q 1/68

(54) **DNA-ANALYSIS METHOD INVOLVING GENE AMPLIFICATION AND MAGNETIC PARTICLES**
DNS-ANALYSE VERFAHREN MIT GENAMPLIFIKATION UND MAGNETISCHEN TEILCHEN
PROCEDE D'ANALYSE D'ADN COMPORTANT UNE AMPLIFICATION GENIQUE ET L'UTILISATION DE PARTICULES MAGNETIQUES

(30) Priority: 24.05.1988 NO 882261
(43) Date of publication of application: 13.03.1991
(73) Proprietor: PAULSEN, Gunnar, N-0265 Oslo 2 (NO); SOLLID, Ludvig M., NO-0177 Oslo 1 (NO); VARTDAL, Frode, N-0387 Oslo 3 (NO)
(72) Inventor: PAULSEN, Gunnar, N-0265 Oslo 2 (NO); SOLLID, Ludvig M., NO-0177 Oslo 1 (NO); VARTDAL, Frode, N-0387 Oslo 3 (NO)
(74) Representative: Dzieglewska, Hanna Eva
(86) International application number: NO8900050
(87) International publication number: WO8911546

(56) References cited:
- EP-A- 204 510
- EP-A- 229 701
- EP-A- 265 244
- WO-A-86/05815
- US-A- 4 672 040
- KJEMI, July 1987; J. AFSETH et al., pp. 63-64#

## Description

The present invention is a new method of gene assaying in humans, animals, plants, and microorganisms. Such assays previously required complicated procedures and time consuming techniques. What is new in this invention is that superparamagnetic particles, e.g. Ugelstad spheres, are used to isolate genes, and that these isolated genes, while attached to the particles, are copied 10⁵-10⁶ times and assayed. Since isolation, copying, and assay occur on the surface of a solid superparamagnetic phase, gene assays are considerably more simple, rapids less labour consuming, and more sensitive. Also, this process may readily be automated.

Solid phases which are commonly used for immobilizing nucleic acids comprise microporous membranes of nitrocellulose or nylon. Hybridization is carried out by immersion of the membrane in a hybridization liquid which contains the complementary nucleic acid sequence. This technique entails a long hybridization period as well as a low degree of hybridization.

Identification of genes may be carried out by probing with complementary DNA sequences (DNA-probes) attached to magnetic particles (cf. EP-A-O 265 244). In restriction fragment length polymorphism assays (RFLP)-assays total DNA is treated in the cells by a restriction endonuclease. Then the fragments are separated electrophoretically and are blotted on filters before probing with a DNA sequence which is complementary to the DNA region of interest. Restriction endonuclease cuts the DNA at palindrome sequences (specific clearing sites of restriction endonucleases) which is in bonding imbalance with allele sequences. Consequently, the size of DNA fragments and, thus, the band patterns may vary. dependent on the allele variations within the region reacting with the utilized DNA-probes. The RFLP technique is a time consuming and complicated method, requiring several days of work for completion. Also, bands vhich are detected in RFLP-blot need not show information of importance, as several restriction sites are common to several alleles. This makes it difficult to evaluate RFLP-blots.

In the polymerase chain reaction technique (PCR) the region of the qenome containing the gene of interest is amplified before probing with oligonucleotides which are complementary to constant or to all polymorphic sequences within the amplified DNA-region (cf. ref. 1,3). This technique substantially reduces the time required for carrying out gene detection assays. But even this method requires extensive handling of the test material, including an amplification step in a test tube, upon which the amplified DNA-material is transferred to a nitrocellulose membrane and analysed with a gene probe.

The object of the present invention is to provide a new method for gene assays in humans, animals, plants, and microorganisms, Where the steps of the assay occur on the surface of a solid superparamagnetic phase, thus, resulting in a gene assay that is more simple and rapid, less labour consuming, and more sensitive.

According to the present invention a method for gene detection assays in a test medium is thus provided, and said method is characterized by
a) denaturing DNA in the test medium,
b) contacting the test medium under hybridizing conditions with heat-resistant DNA-polymerase, deoxynucleotides, two different oligonucleotide primers, one of which is soluble, whereas the other is bonded to superparamagnetic particles,
c) amplification of DNA by the polymerase chain reactions;
d) removal of the DNA strand which is not connected with the particles, as well as free oligonucleide primers,
e) detecting amplified material.

The test medium may be placed in test wells or the wells of a transparent multi-well plate. The multi-well plate is then covered by a lid and is placed in a metal block which is brought into close contact with the lid, bottom, and sides of each well on the plate. The temperature in this metal block may be rapidly and automatically changed. To destroy the cells and denature the DNA, the temperature of the metal block is adjusted to 100°C, and the multi-well plate is incubated at said temperature for 5-10 min. A temperature which depends on the length and the purine/pyrimidine ratio of the oligonucleotide primer is set, and heat resistant DNA polymerase, deoxynucleotides, two different oligonucleotide primers (one soluble and one bonded to superparamagnetic particles) are added to each well. Amplification of DNA is carried out by alternating the temperature of the metal block between a temperature determined by the primer used, e.g. approx. 20-85°C, and 90°C during a number of cycles depending on the degree of amplification required for the test medium to be assayed. The incubation period is 1-2 min. for each cycle at the temperature determined by the used primer, and 0.5 min. at 90°C. The multi-well plate is then placed on the magnet and the wells are washed at 90°C to remove the DNA strand which is not bonded top particles, as well as the free oligonucleotide primers. Amplified material may then be dejected either by (A) specific probing with oligonucleotides and demonstration of labelled nucleotides without, or after reneved polymerase reaction, or (B) specific probing, polymerase reaction, and demonstration of double-strand DNA with labelled intercalating substances, or (C) assayed by DNA sequence assay.
A.
   Specific probing with oligonucleotides may be carried out in three different manners:
A1.
   Probing with labelled oligonucleotide probes may be carried out by adding a biotinylated oligonucleotide probe to each well at a temperature determined by the probe used. The multi-well plate is then incubated at said temperature for 5-10 min. Then the multi-well plate is placed on a magnet and each well is washed. To the biotine labelled probes, which are bonded to amplified DNA, avidine (or streptavidine) connected to enzymes, fluorochromes, etc. is added. Bonding of biotinylated probes is then analyzed by use of relevant detecting systems for the different labels, as chemiluziniscense for detection of peroxidase-labelled avidine. In stead of biotine-labelled probes, radioactive labelled probes may be used.
A2.
   Production of labelled DNA by the aid of specific oligonucleotide primers may be carried out by adding a mixture of non-labelled and biotinylated deoxynucleotides, DNA polymerase, and an oligonucleotide probe to particle-bonded amplified DNA, and incubating at a temperature determined by the length and composition of this probe. Particle-bonded amplified single-strand DNA will serve as a template, whereas the oligonucleotide probe will serve as a primer. In this manner, a DNA strand containing labelled nucleotides will be produced. Remaining steps of the probing method are carried out as described under A1. In stead of biotine-labelled nucleotides, radioactive-labelled probes may be used in the same manner as in conventional techniques.
B.
   The production of DNA by use of a specific oligonucleotide probe and following detection of double-strand DNA by use of intercalating substances may be carried out by adding a mixture of deoxynucleotides, DNA-polymerase, and an oligonucleotide probe to particle-bonded amplified single-strand DNA, and incubating at a temperature determined by the length and nucleotide composition of the probe. Particle bonded single-strand DNA will serve as a template, whereas the oligonucleotide probe may serve as a primer and synthesis of the DNA-strand complementary to the template will occur. This double-strand DNA may now be demonstrated by adding a biotinylated compound which intercalates non-specifically with double-strand DNA.

The remaining steps of detection are carried out as mentioned under A1.
C. Sequence analysis
   The superparamagnetic spheres with bonded amplified DNA are heated to 95°C for 5 minutes and washed, and DNA-polymerase, and soluble primer are added once more (this time, e.g. labelled 32P), and the dideoxy method (ref. 2) may be Used for sequencing. Before analysis the sample is heated for some minutes, e.g. for 2 min. to 95°C, and DNA bonded to superparamagnetic spheres is removed by the sid of a magnet. The sequence analysis per se, e.g. by polyacrylamid gel electrophoresis, is in this case carried out on the soluble fraction.

The fundamentally new feature of the present invention is that superparamagnetic particles bonded to oligo-nucleotides are at the same time used to (1) isolate (catch) relevant DNA sequences from various biological matter (eukaryotes) or microorganisms, (2) as a solid phase on which DNA is amplified by the polymerase chain reaction, and (3) as a solid carrier on which detection of amplified material can be carried out by specific probing with use of labelled allele or locus-specific oligonucleotides which may also function as primers for a polymerase reaction for further incorporation of labelled nucleotides and, consequently, enhanced sensitivity, or in demonstration of double-strand DNA with intercalating substances. For the above mentioned methods of detection the specificity of the gene assay may be mediated both by the oligonucleotide primers on the particles, since amplification of DNA is only achieved after base pairing of oligonucleotide primers to complementary sequences, and in the probing step.

The advantages of the present invention are many. Bonding of the amplified DNA material to superparamagnetic particles permits both probing and analysis to be carried out in the same container which was used for amplification and isolation of the DNA. This will reduce handling and transfer of the test medium between different containers during production and permit simple and automatic production of the test medium. Since the amplified DNA is bonded to the surface of superparamagnetic particles, washing in the steps of catching, probing, and detection can be carried out readily, efficiently, and very quickly by use of a magnet, which permits complete automation of the entire asssay.

Probed amplified material on the particles may be focused in a small spot on the bottom of the U-shaped micro-wellplate in less than a second by utilizing a magnet. This will result in much improved sensitivity of the assay system, since the intensity of liberated energy per mm will increase when the surface of the spot is reduced.

A DNA strand is amplified on the surface of magnetic particles, and this strand which may readily be separated from the non-bonded strand after PCR-amplification may then be used as a template for synthesis of a biotinylated DNA strand initiated after specific annealing of an allele or locus specific oligonucleotide probe complementary to the particle bonded DNA strand. The degree of labelling and the sensitivity of the analysis are, thus, greatly enhanced. By using particles bonded to oligonucleotides, only relevant DNA is amplified and isolated. This is specially advantageous when the technique is used to identify infectious DNA (like virus and bacteria) in biological liquids or tissue from eukaryotic organisms.

It is, furthermore underlined that the above disclosed method for isolating single-strand DNA may also be used for sequenee analysis.

DNA sequence analysis requires a series of operational and time consuming steps before the sequence analysis proper. They comprise introduction of a DNA sequence of interest, e.g. in plasmids, infection by bacteria of the latter, cloning of bacteria, expansion of bacteria clones, purification of plasmid from the same, "cutting out" the DNA sequence of interest by restriction enzymes, purification of this DNA sequence which may then be sequence analysed. By amplification using superparamagnetic spheres the labour consuming steps carried out before sequence analysis proper can be omitted.
- Ref. 1:: R.K. Saiki, T.L. Bugavan, G.T. Horn, K.B. Mullis and H.H. Erlich. Nature 324, 163.166, 1986.
- Ref. 2:: F. Sanger, S. Miklen and A.R. Coulson. Proc. Nat. Acad. Sci. USA. 74, 5463-5467, 1977.
- Ref. 3:: R.K. Saiki, D.H. Gelfand, S. Stoffel, S.J. Scharf, R. Higuchi, G.T. Horn, K.B. Mullis & H.A. Erlich. Nature 239, 487-491, 1988.

## Claims

1. A method for examination of genes in a test medium,
**characterized in** that it comprises:
a) denaturing DNA in the test medium,
b) contacting the test medium in hybridizing conditions with heat resistant DNA-polymerase, deoxynucleotides, two different oligonucleotide primers, one of which is soluble, whereas the other is bonded to superparamagnetic particles,
c) amplification of DNA by the polymerase chain reaction,
d) removal of the DNA strand which is not bonded to the particles, as well as free oligonucleotide primers,
e) detecting amplified material.

2. A method as stated in claim 1, **characterized in** that the DNA is denatured at a temperature in the region of 95-105°C and for a period of 5-10 minutes.

3. A method as stated in claim 1, **characterized in** that the amplification temperature is determined by the length and nucleotide composition of the primers.

4. A method as stated in claim 1, **characterized in** that the DNA is amplified in a number of cycles by alternating the temperature during a period of 1-2 minutes at amplification temperature and for 0.5 minutes at 85-95°C.

5. A method as stated in claim 1, **characterized in** that after heating for approximately 2 minutes to 95°C, the DNA strand-which is not bonded to the particles, as well as free oligonucleotide primers are removed by washing by use of a magnet.

6. A method as stated in claim 1, **characterized in** that the amplified material is detected with a specific labelled probe.

7. A method as stated in claim 1, **characterized in** that upon amplification of DNA, removal and detection under items (d) and (e) are carried out by addition of a soluble primer, a mixture of dideoxynucleotides and deoxynucleotides and a DNA-polymerase and the reaction is permitted to go on for some minutes, heating is carried out for approximately 2 minutes at 95°C, the superparamagnetic particles with their DNA are removed, and DNA sequence analysis of the soluble material is carried out.

8. A method as stated in claim 1, 2, and 6, **characterized in** that after denaturing DNA in the test medium, the test medium is contacted under hybridizing conditions with heat resistant DNA polymerase, deoxynucleotide and two different soluble oligonucleotide primers and the DNA is amplified by the polymerase chain reaction, upon which superparamagnetic particles carrying a specific primer are added and the polymerase reaction is carried out at least once, followed by washing by use of magnet, denaturing, and adding labelled deoxynucleotides, soluble primer, heat resistant DNA polymerase, amplifying by the polymerase chain reaction, denaturing, washing by use cf magnet and detecting labelled single-strand DNA on the spheres.

## Patentansprüche

1. Verfahren zur Untersuchung von Genen in einem Test-Medium, dadurch gekennzeichnet, daß es umfaßt:
a) Denaturieren von DNA in dem Test-Medium,
b) Inkontakt bringen des Testmediums unter Hybridisierungsbedingungen mit hitzestabiler DNA-Polymerase, Desoxynucleotiden, 2 unterschiedlichen oligonucleotid-Primern, von denen einer löslich ist und der andere an superparamagnetische Teilchen gebunden ist,
c) Amplifizieren von DNA mittels der Polymerase-Kettenreaktion,
d) Entfernen des nicht an die Teilchen gebundenen DNAStranges sowie freier oligonucleotid-Primer,
e) Nachweis von amplifiziertem Material.

2. Verfahren nach Anspruch 1, dadurch gekennzeichnet, daß die DNA bei einer Temperatur im Bereich von 95°-105°C und für eine Zeitspanne von 5-10 Minuten denaturiert wird.

3. Verfahren nach Anspruch 1, dadurch gekennzeichnet, daß die Amplifizierungs-Temperatur nach der Länge und der Nucleotid-Zusammensetzung der Primer bestimmt wird.

4. Verfahren nach Anspruch 1, dadurch gekennzeichnet, daß die DNA in einer Reihe von Zyklen durch Alternieren der Temperatur für eine Zeitspanne von 1-2 Minuten bei Amplifizierungs-Temperatur und für 0,5 Minuten bei 85-95°C amplifiziert wird.

5. Verfahren nach Anspruch 1, dadurch gekennzeichnet, daß, nach Erhitzen für etwa 2 Minuten auf 95°C, der nicht an die Teilchen gebundene DNA-Strang sowie freie oligonucleotid-Primer durch Waschen unter Verwendung eines Magneten entfernt werden.

6. Verfahren nach Anspruch 1, dadurch gekennzeichnet, daß das amplifizierte Material mit einer spezifischen markierten Sonde nachgewiesen wird.

7. Verfahren nach Anspruch 1, dadurch gekennzeichnet, daß, nach Amplifizieren der DNA, die Entfernung und der Nachweis gemäß Punkt (d) und (e) durchgeführt wird, indem ein löslicher Primer, ein Gemisch von Didesoxynucleotiden und desoxynucleotiden und eine DNA-Polymerase zugesetzt und die Reaktion einige Minuten ablaufen gelassen wird, für etwa 2 Minuten auf 95°C erhitzt wird, die superparamagnetischen Teilchen mit ihrer DNA entfernt werden und die DNA-Sequenzanalyse des löslichen Materials durchgeführt wird.

8. Verfahren nach Anspruch 1, 2 und 6, dadurch gekennzeichnet, daß, nach Denaturieren von DNA in dem Testmedium, das Testmedium unter Hybridisierungsbedingungen mit hitzestabiler DNA-Polymerase, Desoxynucleotid und zwei unterschiedlichen, löslichen oligonucleotid-Primern in Kontakt gebracht wird und die DNA durch die Polymerase-Kettenreaktion amplifiziert wird, worauf superparamagnetische Teilchen, die einen spezifischen Primer tragen, zugesetzt werden, und die Polymerase-Reaktion mindestens einmal durchgeführt wird, gefolgt von Waschen unter Verwendung eines Magneten, Denaturieren, und Zugabe von markierten Desoxynucleotiden, löslichem Primer, hitzestabiler DNA-Polymerase, Amplifizieren mittels Polymerase-Kettenreaktion, Denaturieren, Waschen unter Verwendung eines Magneten und Nachweis markierter einzelsträngiger DNA auf den Kügelchen.

## Revendications

1. Procédé de recherche de gênes dans un milieu, caractérisé en ce qu'il comprend :
a) la dénaturation de l'ADN dans le milieu,
b) la mise en contact du milieu dans des conditions d'hybridation avec une ADN-polymérase thermostable, des désoxynucléotides, et deux amorces oligonucléotidiques différentes, l'une étant soluble et l'autre étant liée à des particules superparamagnétiques,
c) l'amplification de l'ADN par PCR,
d) l'élimination du brin d'ADN qui n'est pas lié aux particules, ainsi que des amorces oligonucléotidiques libres,
e) la détection du matériel amplifié.

2. Procédé selon la revendication 1, caractérisé en ce que l'ADN est dénaturé à une température de 95-105° C et pendant 5-10 minutes.

3. Procédé selon la revendication 1, caractérisé en ce que la température d'amplification est déterminée par la longueur et la composition en nucléotides des amorces.

4. Procédé selon la revendication 1, caractérisé en ce que l'ADN est amplifié dans une série de cycles avec alternance des températures, à savoir pendant une période 1 à 2 minutes à la température d'amplification et pendant 0,5 minutes à 85-95° C.

5. Procédé selon la revendication 1, caractérisé en ce qu'après chauffage pendant environ 2 minutes à 95° C, le brin d'ADN qui n'est pas lié aux particules ainsi que les amorces oligonucléotidiques libres sont éliminés par lavage avec utilisation d'un aimant.

6. Procédé selon la revendication 1, caractérisé en ce que le matériel amplifié est détecté avec une sonde marquée spécifique.

7. Procédé selon la revendication 1, caractérisé en ce qu'après amplification de l'ADN, l'élimination et la détection selon les stades d) et e) sont effectuées par addition d'une amorce soluble, d'un mélange de didésoxynucléotides et désoxynucléotides et une ADN-polymérase et la réaction est laissée s'effectuer pendant quelques minutes, un chauffage est effectué pendant environ 2 minutes à 95° C, les particules superparamagnétiques avec leur ADN sont éliminées et une analyse de la séquence d'ADN du matériel soluble est effectuée.

8. Procédé selon les revendications 1, 2 et 6, caractérisé en ce qu'après dénaturation de l'ADN dans le milieu, le milieu est mis en contact dans des conditions d'hybridation avec une ADN-polymérase thermostable, des désoxynucléotides et deux amorces oligonucléotidiques solubles différentes et l'ADN est amplifié par PCR, puis des particules superparamagnétiques portant une amorce spécifique sont ajoutées et la réaction avec la polymérase est effectuée au moins une fois, cette réaction étant suivie d'un lavage avec utilisation d'un aimant, d'une dénaturation et de l'addition de désoxynucléotides marqués, d'une amorce soluble et d'ADN-polymérase thermostable, d'une amplification par PCR, d'une dénaturation, d'un lavage avec utilisation d'un aimant et de la détection de l'ADN monobrin marqué sur les sphères.
